Europäisches Patentamt

(19) European Patent Office

Office européen des brevets

(11) Publication number: **0 454 390 A1**

## (12) EUROPEAN PATENT APPLICATION

(21) Application number: **91303578.8**

(51) Int. Cl.⁵: **A61M 16/18**

(22) Date of filing: **22.04.91**

(30) Priority: **25.04.90 GB 9009271**

(43) Date of publication of application:
**30.10.91 Bulletin 91/44**

(84) Designated Contracting States:
**DE FR GB IT**

(71) Applicant: **The BOC Group plc**
**Chertsey Road**
**Windlesham Surrey GU20 6HJ (GB)**

(72) Inventor: **Montgomery, Frederick John**
**34 Birchlands Grove**
**Wilsden, Bradford BD15 0HD (GB)**

(74) Representative: **Belcher, Simon James et al**
**Urquhart-Dykes & Lord Tower House Merrion**
**Way**
**Leeds LS2 8PA (GB)**

(54) Anaesthetic vaporiser.

(57) An anaesthetic vaporiser 1 for use particularly with anaesthetic agents having a low boiling point, comprises an inlet 2 for carrier gas and an outlet 4 for carrier gas and gaseous anaesthetic agent. A first passage 6 extends between the inlet and the outlet. A flow restrictor 8 is located within the first passage 6. A second passage 10 extends between the first passage 6 at a location upstream of the flow restrictor 8 and a regulator 14. The regulator 14 controls the pressure of gaseous anaesthetic agent when flowing through a third passage 26, extending from a vaporising chamber 12 containing anaesthetic agent to the first passage 6 at a location downstream of the flow restrictor in the first passage. A flow control valve 28 is located in the third passage 26. A fourth passage extends from a source of carrier gas to the base of the vaporising chamber 12 to allow bubbles of carrier gas to pass through liquid anaesthetic agent when contained in the vaporising chamber.

FIG 1

The present invention relates to an anaesthetic vaporiser.

An anaesthetic vaporiser of the by-pass type is disclosed in GB-1224478. In that vaporiser, a carrier gas such as oxygen, air or nitrous oxide is initially divided on entry to the vaporiser between a first stream which is directed towards the sump or vaporising chamber of the vaporiser to entrain vapour from a volatile liquid anaesthetic contained therein; and a second by-pass stream, the first and second streams subsequently recombining prior to leaving the vaporiser for delivery to a patient.

This known vaporiser has been used successfully over a number of years for delivering anaesthetic agents such as halothane, trichloroethylene and ether derivatives including enflurane, fluoroxene, methoxyflurane and isoflurane. All the aforementioned anaesthetic agents have a boiling point at atmospheric pressure well in excess of 40°C.

A new anaesthetic agent, 2-(difluoromethoxy)-1,1,1,2-tetrafluoroethane, has been developed which has a boiling point at normal atmospheric pressure characteristic of about 20 to 25°C. This physical characteristic makes vaporisers of the type disclosed in GB-1224478 unsuitable for delivering 2-(difluoromethoxy)-1,1,1,2-tetrafluoroethane to a patient, since the boiling point is approximately in the middle of the normal operating temperature range of such a vaporiser, which is generally about 15 to 35°C. When the ambient temperature, and hence the vaporiser temperature, is above 25°C, latent heat of vaporisation transferred to the low boiling point anaesthetic agent and causes an amount of the agent to vaporise until the heat lost to the latent heat of vaporisation is equal to the heat transferred to the agent.

The present invention seeks to provide an anaesthetic vaporiser which is capable of delivering to a patient a predetermined concentration of an anaesthetic agent having a low boiling point, for example less than about 30°C, such as about 20°C.

Accordingly, in one aspect, the invention provides an anaesthetic vaporiser, which comprises:

(a) an inlet for carrier gas;

(b) an outlet for carrier gas and gaseous anaesthetic agent for delivery to a patient;

(c) a first passage extending between the inlet and the outlet;

(d) a flow restrictor located in the first passage;

(e) a vaporising chamber for anaesthetic agent;

(f) a first regulator for controlling the pressure of anaesthetic agent vapour when flowing from the vaporising chamber to the outlet;

(g) a second passage extending between the first passage at a location upstream of the flow restrictor and the first regulator;

(h) a third passage extending from the vaporising chamber to the first passage at a location downstream of the flow restrictor, through which anaesthetic agent vapour can flow from the chamber towards the outlet; and

(i) a flow control valve located in the third passage.

The inclusion of a regulator in the vaporiser of the invention, connected to the carrier gas inlet by a passage, has the advantage that it makes it possible for the concentration of the anaesthetic agent in carrier gas supplied through the inlet to remain constant, substantially irrespective of the rate of flow of the carrier gas. The concentration of the anaesthetic agent in the carrier gas can be set by the flow control valve in the third passage, which is preferably a laminar flow device, and manually adjustable.

The vaporiser may include a fourth passage which extends from a source of carrier gas to the base of the vaporising chamber, the fourth passage terminating at a location coincident with a bubbling structure located within the vaporising chamber such that carrier gas when passed along the fourth passage enters the vaporising chamber and is formed into bubbles which pass up through liquid anaesthetic agent when contained within the chamber. This makes it possible to use the vaporiser of the invention to be used with an anaesthetic agent without a source of heat for that agent by which the agent might otherwise be vaporised.

The restrictor placed in the first passage will commonly be a laminar flow device. In some circumstances, it can be advantageous to use a thermally responsive valve, by which adjustment can be made for temperature effects.

The vaporiser may include a source of heat for the anaesthetic agent within the vaporising chamber, or within any of the first, second and third passages, or both, by which, for example, the temperature of the agent can be maintained at a predetermined level relative to the boiling point of the agent.

The vaporiser may include a further first regulator for controlling the pressure of gaseous anaesthetic agent when flowing through the third passage, provided with a further passage which extends between the first passage at a location upstream of the flow restrictor therein, and the said further first regulator. The provision of a further first regulator increases the level of safety of the vaporiser, as a backup for the primary first regulator.

The vaporiser may include a by-pass passage extending from the first passage adjacent the inlet to the vaporising chamber, a one-way valve located in the by-pass passage, and a back pressure regulator located in the first passage. These features can allow the vaporiser of the invention to be used to supply to a patient an anaesthetic agent whose boiling point is above the normal operating temperature of the vaporiser, for example above 20°C when the normal operating temperature range is in the order of 15 to 35°C. When the operating temperature of the vaporiser is above the boiling point of the anaesthetic agent, the one-way valve will be closed; when the operating temperature is below the boiling point, the one-way valve opens and the vaporiser is able to perform as a normal vaporiser of the by-pass type, in which the laminar flow restrictor in the first passage is controlled dependent on the operating temperature.

Embodiments of the invention will now be described, by way of example, reference being made to the Figures of the accompanying diagrammatic drawings in which:

Figure 1 is a diagrammatic sketch of an anaesthetic vaporiser according to the present invention in which are used ISO/BSI symbols;

Figure 2 is a side view partly in cross section of the anaesthetic vaporiser of Figure 1 but with the addition of heaters as illustrated in Figure 4;

Figure 3 is a diagrammatic sketch of a modified version of the anaesthetic vaporiser of Figure 1;

Figure 4 is a diagrammatic sketch of a further modified version of the anaesthetic vaporiser shown in Figure 1;

Figure 5 is a diagrammatic sketch of another modified version of the anaesthetic vaporiser shown in Figure 1;

Figure 6 is a diagrammatic sketch of yet a further modified anaesthetic vaporiser as shown in Figure 1; and

Figure 7 is a diagrammatic sketch of still a further modification of the anaesthetic vaporiser of Figure 1.

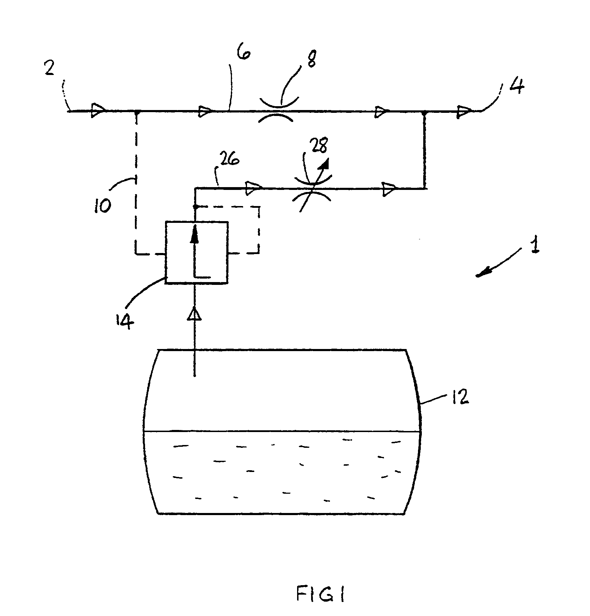

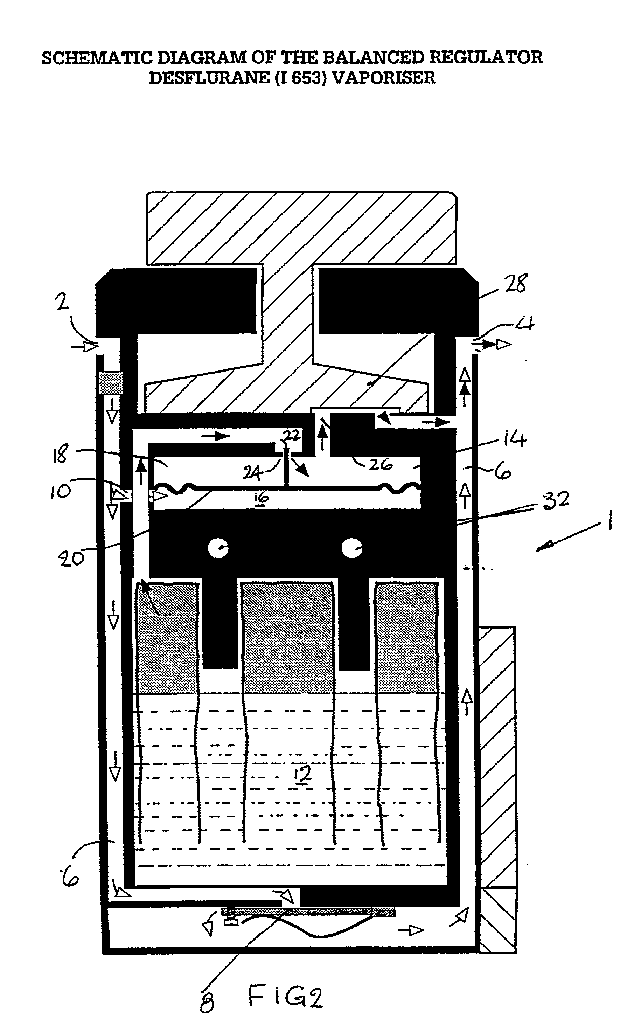

Referring first to Figures 1 and 2, an anaesthetic vaporiser 1 has an inlet 2 for carrier gas and an outlet 4 for carrier gas and gaseous anaesthetic agent. Extending between the inlet 2 and outlet 4 is a passage 6 in which is located a laminar flow by-pass restrictor 8. The restrictor 8 exhibits laminar flow characteristics over its operating flow range.

Extending from the passage 6 from a location upstream of the restrictor 8 is a second passage 10 communicating with a first chamber 16 of a balance regulator 14. The balance regulator 14 includes a second chamber 18 which is separated from the first chamber 16 by diaphragm 20. Connected to the diaphragm 20 for movement therewith is a valve head 22 which co-operates with a valve seat 24. The valve head 22 and valve seat 24 control the flow of gaseous anaesthetic agent contained in a vaporising chamber 12.

Extending from the second chamber 18 is a passage 26 which extends to the passage 6 downstream of the restrictor 8. A laminar flow control valve 28 is located in the passage 26.

The vaporiser 1 includes heaters 32 which are controlled by a temperature sensing device (not shown) However, the heaters 32 can, in a modification, be controlled by a device for sensing the pressure of anaesthetic agent in the vaporising chamber 12.

In use, energy is supplied to the heaters 32 which converts anaesthetic agent from a liquid to a gaseous state which is contained in the upper (as shown) portion of the vaporising chamber 12. Carrier gas then enters inlet 2 and continues along passage 6 through restrictor 8 towards the outlet 4. The pressure upstream of the restrictor 8 is dependent on the flow rate of carrier gas entering the inlet 2. The pressure in the first chamber 16 of the balance regulator 14 is the same as that upstream of restrictor 8 because of the second passage 10. An increase in carrier gas pressure at the inlet 2 causes the diaphragm 20 to move upwards (as shown) taking with it the valve head 22. The valve head 22 will thus separate from the valve seat 24 thereby enabling gaseous anaesthetic agent to leave the vaporising chamber 12 and pass through the second chamber 18 into the passage 26 until the pressure in the passage 26 is the same as that in the chamber 16. The pressure in passage 6 upstream of the restrictor 8 and passage 26 upstream of the control valve 28 are the same. For any position of the control valve 28 the flow rate of gaseous anaesthetic agent will depend on that pressure and hence the carrier gas flow rate at the inlet 2. This ensures that the flow of anaesthetic agent rises when the carrier gas flow rate rises and vice versa and hence the percentage concentration by volume of the anaesthetic agent in the gas delivered to the patient remains constant.

The gaseous anaesthetic agent then joins the carrier gas in the passage 6 prior to leaving the vaporiser outlet 4.

As is known in the art and as explained above the concentration of gaseous anaesthetic agent in the carrier gas leaving the outlet 4 is controlled by the setting of the laminar control valve 28.

The profiles of the valve head 22 and the cooperating valve seat 24 are so shaped that at carrier gas flows of up to 15 litres per minute the pressure of gaseous anaesthetic agent in the passage 26 equals the pressure of the carrier gas entering the inlet 2.

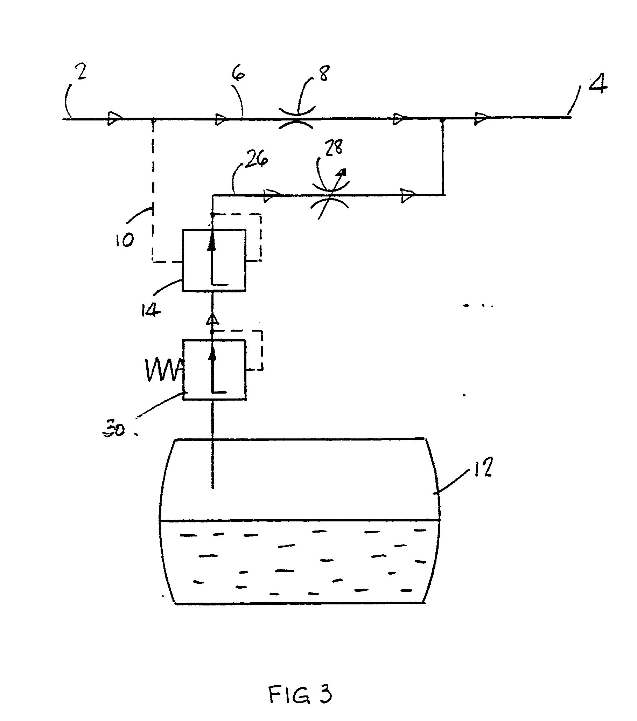

Now referring to Figures 3 to 7 where like reference numerals denote like features as those described with reference to Figures 1 and 2.

Referring in particular to Figure 3, for very low boiling point anaesthetic agents such as cyclopropane with a boiling point of minus 32°C the pressure of the agent in the vaporising chamber 12 at 22°C can be very high, in the order of 75 psi (52.7 g.mm$^{-2}$). In order to cope with this high pressure a second pressure reducing regulator 30 is located between the vaporising chamber 12 and the balance regulator 14.

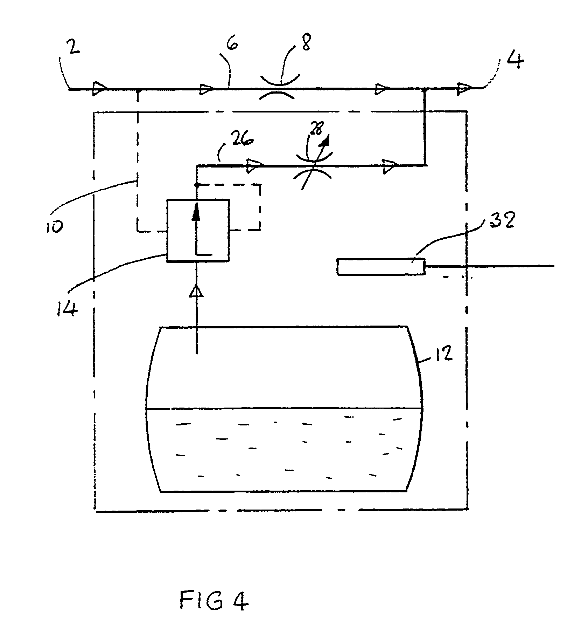

Referring in particular to Figure 4, when the boiling point of the anaesthetic agent is about ambient temperature then to ensure pressure in the vaporising chamber 12 a heater 32 is provided to raise the temperature of the anaesthetic agent to above its boiling point. In the embodiment illustrated in Figure 4 the heater 32 is positioned within the vaporiser such that, as well as the vaporising chamber 12, the passages 6, 10, 26 and regulator 14 are also heated to stop the anaesthetic condensing out on the walls of the vaporiser which would otherwise be cooler than the vaporising chamber.

The heater 32 is controlled by a temperature sensing device (not shown).

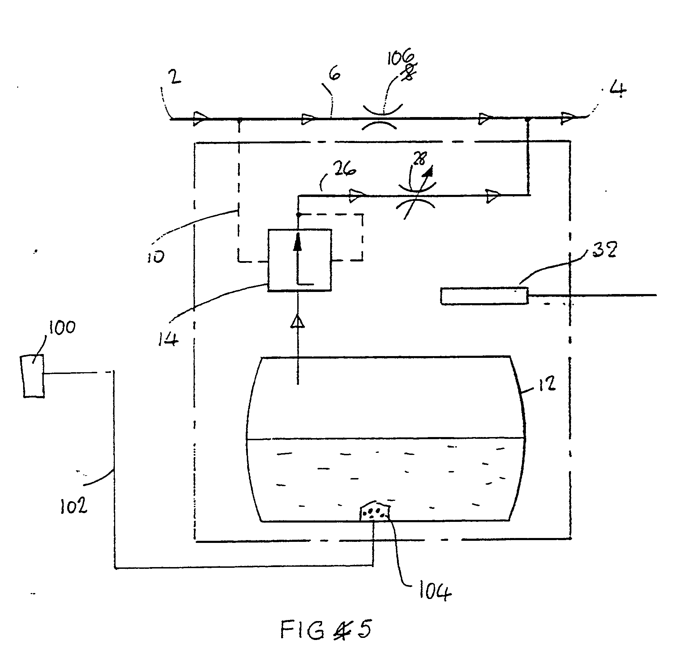

In Figure 5 there is illustrated a modification of the anaesthetic vaporiser 1 shown in Figure 4 in that the heater 32 is no longer available and a need arises to vaporise the liquid anaesthetic agent contained within the vaporising chamber 12 by some other means.

As shown, oxygen or some other carrier gas is supplied from a source 100 and is regulated to flow along a passage 102 at approximately 5 psi (3.5 g.mm$^{-2}$). The passage 102 enters the base of the vaporising chamber 12 at a position coincident with a bubbling structure 104, for example, a honeycomb device, located within the vaporising chamber 12.

It will be apparent, that when carrier gas passes through the passage 102 and into the vaporising chamber 12 minute bubbles of carrier gas will pass through the liquid anaesthetic agent contained within the vaporising chamber thereby vaporising said liquid anaesthetic agent.

It has been found that the concentration of anaesthetic agent in the gas mixture in the vaporising chamber 12 immediately above the liquid anaesthetic agent is approximately 70% by volume.

A further modification shown in Figure 5 is that the fixed restrictor 8 is replaced by a thermally responsive valve 106 to allow for adjustment for temperature affects.

The modifications shown in Figure 5 are particularly important when, for example, the anaesthetic vaporiser 1 is required for use at locations when no electrical power is available to operate a heater 32.

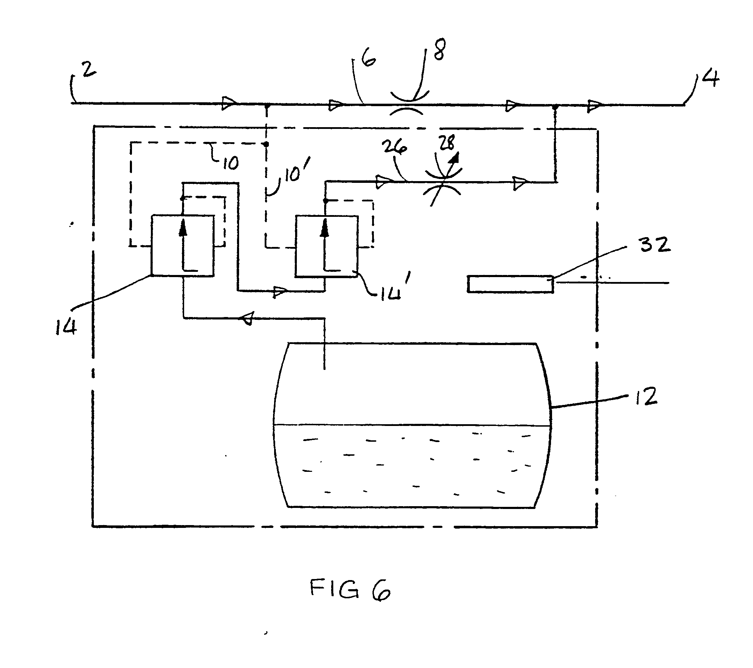

In Figure 6 there is illustrated a modification which provides additional safety in case the regulator 14 should fail in that it provides for a second regulator 14′ in series with the regulator 14.

As shown, extending from the passage 6 is a passage 10 to the regulator 14 and a separate passage 10′ to the regulator 14′.

A further additional safety measure is the provision of a pressure device (not shown) arranged between the passages 6 and 26 which is arranged to monitor the performance of the regulator 14. The pressure device will automatically detect, for example, a situation where the regulator valve head 22 jams open and higher concentrations of anaesthetic agent than those set are being delivered to the outlet 4.

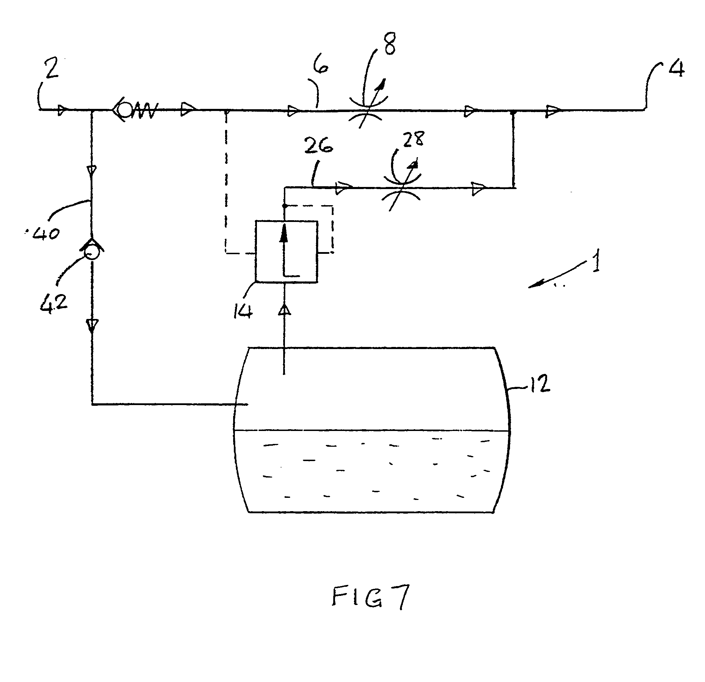

Finally, referring to Figure 7, the vaporiser 1 includes a by-pass passage 40 extending from the passage 6 adjacent the inlet 2 to the vaporising chamber 12. A one-way valve 42 is located in the passage 40. Further, a constant back pressure restrictor 44 is located in the passage 6 as shown. When it is necessary to use anaesthetic agents whose boiling points are above ambient, for example, above 200°C within normal operating temperature ranges in the order of 150°C to 350°C then when ambient temperature is above the boiling point, the vaporiser acts as in the embodiment illustrated in Figures 1 and 2 with the one-way valve 42 closed. When the ambient temperature is below the boiling point of the anaesthetic agent the one-way valve 42 opens and the vaporiser acts as a normal vaporiser of the by-pass type with the laminar flow restrictor 8 being temperature controlled. The laminar flow restrictor 8 thus compensates for changing vapour pressure.

## Claims

1. An anaesthetic vaporiser, which comprises:
   (a) an inlet (2) for carrier gas;
   (b) an outlet (4) for carrier gas and gaseous anaesthetic agent for delivery to a patient;
   (c) a first passage (6) extending between the inlet and the outlet;
   (d) a flow restrictor (8) located in the first passage;
   (e) a vaporising chamber (12) for anaesthetic agent;
   (f) a first regulator (14) for controlling the pressure of anaesthetic agent vapour when flowing from the vaporising chamber to the outlet;
   (g) a second passage (10) extending between the first passage at a location upstream of the flow restrictor and the first regulator;
   (h) a third passage (26) extending from the vaporising chamber to the first passage at a location downstream of the flow restrictor, through which anaesthetic agent vapour can flow from the chamber towards the outlet; and
   (i) a flow control valve (28) located in the third passage.

2. A vaporiser as claimed in claim 1, in which the flow restrictor (8) and the flow control valve (28) are substantially laminar flow devices over their operating flow ranges.

3. A vaporiser as claimed in claim 1 or claim 2, which includes a fourth passage (102) which extends from a source (100) of carrier gas to the base of the vaporising chamber (12), the fourth passage terminating at a location coincident with a bubbling structure (104) located within the vaporising chamber such that carrier gas when passed along the fourth passage enters the vaporising chamber and is formed into bubbles which pass up through liquid anaesthetic agent when contained within the chamber.

4.  A vaporiser as claimed in claim 3, in which the flow restrictor (106) in the first passage is in the from of a thermally responsive valve.

5.  A vaporiser as claimed in any one of claims 1 to 4, which includes a pressure reducing regulator (30) located between the vaporising chamber (12) and the said first regulator (14).

6.  A vaporiser as claimed in any one of claims 1 to 5, which includes a source of heat (32) for the anaesthetic agent within the vaporising chamber (12).

7.  A vaporiser as claimed in any one of claims 1 to 6, which includes a source of heat (32) for the anaesthetic agent within any of the first, second and third passages (6, 10, 26).

8.  A vaporiser as claimed in claim 6 or claim 7, in which the or each source of heat is controlled by a temperature sensing device.

9.  A vaporiser as claimed in claim 6 or claim 7, in which the or each source of heat is controlled by a device by which the pressure of anaesthetic agent vapour in the vaporising chamber can be monitored.

10.  A vaporiser as claimed in any one of claims 1 to 9, which includes a further first regulator (14') for controlling the pressure of gaseous anaesthetic agent when flowing through the third passage (26), provided with a further passage (10')which extends between the first passage (6) at a location upstream of the flow restrictor (8) therein, and the said further first regulator.

11.  A vaporiser as claimed in any one of claims 1 to 10, which includes a by-pass passage (40) extending from the first passage (6) adjacent the inlet (2) to the vaporising chamber (12), a one-way valve (42) located in the by-pass passage, and a back pressure restrictor (44) located in the first passage (6).

FIG 1

SCHEMATIC DIAGRAM OF THE BALANCED REGULATOR
DESFLURANE (I 653) VAPORISER

FIG2

FIG 3

FIG 4

FIG 45

FIG 6

FIG 7

European Patent
Office

**EUROPEAN SEARCH REPORT**

Application Number

EP    91 30 3578

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | DE-A-1 917 774 (VEB MEDIZINTECHNIK LEIPZIG) <br> * page 3, line 24 - page 4, line 13; figure 1 * | 1 | A61M16/18 |
| A | US-A-4 881 541 (EGER,II ET AL) <br> * abstract; figures 1,2 * <br> * column 2, line 29 - column 3, line 30 * | 1 | |
| A | EP-A-231 513 (GAMBRO ENGSTRÖM AB) <br> * abstract; figures * | 1 | |
| A | BRITISH JOURNAL OF ANAESTHESIA <br> vol. 34, no. 10, 1962, <br> pages 741 - 744; <br> W. EDMONDSON & D.W. HIL: 'A PRESSURIZING VALVE FOR THE FLUOTEC VAPORIZER ' <br> * page 742, left column, line 5 - right column, line 16; figures 1,2; tables 1,2 * <br> * page 744, right column, line 6 - line 17 * | 1 | |
| A | DE-A-3 315 943 (DRÄGERWERK AG) <br> * abstract; figure 1 * | 1 | TECHNICAL FIELDS SEARCHED (Int. Cl.5) <br><br> A61M |

The present search report has been drawn up for all claims

| Place of search | Date of completion of the search | Examiner |
|---|---|---|
| THE HAGUE | 26 JULY 1991 | ZEINSTRA H. |

EPO FORM 1503 03.82 (P0401)